# EUROPEAN PATENT SPECIFICATION

(11) **EP 1 711 062 B1**
(45) Date of publication and mention of the grant of the patent: **01.12.2010**
(21) Application number: 04802157.0
(22) Date of filing: 22.12.2004
(51) Int. Cl.: A21D 2/00, A21D 8/04, C12N 1/16

(54) **LIQUID LEAVEN COMPOSITION**
FLÜSSIGE TEIGLOCKERUNGSMITTELZUSAMMENSETZUNG
Composition de lévain liquide

(30) Priority: 22.12.2003 EP 03447304
(43) Date of publication of application: 18.10.2006
(73) Proprietor: PURATOS N.V., 1702 Groot-Bijgaarden (BE)
(72) Inventor: BONJEAN, Bernard, B-1495 Marbais (BE); CAPPELLE, Stefan, B-9500 Onkerzele (BE); DEWILDE, Christophe, Mount Laurel, NJ 08054 (US); TOSSUT, Pierre Patrick Aldo, B-4350 Remicourt (BE)
(74) Representative: pronovem
(86) International application number: PCT/BE2004/000181
(87) International publication number: WO 2005/060757

(56) References cited:
- EP-A- 0 153 057
- EP-A- 1 206 913
- WO-A-91/12315
- WO-A-03/048342
- US-A- 3 174 867
- US-A- 4 211 798

## Description

### Field of the invention

The present invention relates to novel stable liquid leaven compositions for bakery, snack (e.g. crackers, pretzels, biscuits, ...) and pizza applications comprising a bread flavour improvement composition, an active yeast and a bread improver composition, to their use and their production.

### Background of the invention

Currently, a bread flavour improvement system, a bread improver composition and the yeast are dosed each separately. There are several disadvantages coupled therewith.

Separate dosing increases the risk of faults as well as the labor costs incurred with said dosing. In case of liquid products, the investments in separate dosing are further significantly higher compared to a single dosing system since all piping, all pumps and automation has to be multiplied by the number of separate dosing points.

Powdered dosing systems on the other hand are less accurate, or they require huge investments for automation. The dust production due to the dosing of powdered products is an increasing problem in the bakery due to its allergic properties.

In terms of waste disposal, separate packaging will generate much more waste compared to an all-in-one solution. Separate products increase the number of stock keeping units, complicating the logistic organization of the bakery as well as a decreased working capital due to immobilization of capital into stocks.

A number of processes are known to improve the quality and flavour of the bread.

Sourdough production and sponge production are two well-known examples of fermentation systems that are produced in a bakery.

The making of house-made sourdough implies a lot of organizational efforts as well as the risk of decreased consistency in the bakery.

For these reasons, ready-to-use sourdough based compositions have been developed and marketed. Liquid sourdough based compositions have entered the market as bakery ingredients.

It is a new trend in bakery to supply the bread improvement system in liquid form. This bread improvement system comprises chemical additives (dough conditioners) such as oxidizing and reducing agents, emulsifiers, fatty materials and others, and enzymes.

Active yeast can be supplied to bakery as liquid product enabling accurate dosing, easier cleaning of the system etc. Liquid yeast can be stabilized by adding hydrocolloids or gums such as xhantane gums or by continuous mixing (see EP-A-0461725). Alternatively, stabilization of the yeast can be obtained by using a 1% exopolysaccharide such as a dextran in the final product thereby preventing decantation as described in US Patent No. 6,399,119.
Document WO-A-03/048342 discloses a composition comprising between 24% and 45% yeast (based on dry matter content) **characterized in that** it contains more than 0.75% salt, in that the composition is liquid and is biologically stable by maintaining the composition below 10°C.
Document EP-A-1 206 913 is related to a composition being a flavoured bread improver comprising an emulsifier, an encapsulated matrix and a co-emulsifier consisting of a blend of aroma chemicals having co-emulsifier properties.
Document EP-A-0 153 057 discloses a sponge-dough process wherein a milled grain substrate in the form of an aqueous slurry is continuously fermented by yeast that is preferably immobilized, in the presence of lactic acid producing bacteria.
Document US 3,174,867 discloses a method for making white bread continuously wherein specific enzyme peroxidised oils are added in particular proportions in a preliminary mixture to provide a flavor improvement in the finished products.

### Aims of the invention

The present invention aims to provide a liquid leaven composition that does not have the drawbacks of the prior art.

It is an aim to provide a liquid leaven composition that is ready to use.

It is another aim to provide a liquid leaven composition that advantageously has the same gassing power as fresh yeast, the dough and bread improvement properties of a regular bread improvement system and flavour enhancement properties as one can achieve with a sourdough process or a sponge process.

It is yet another aim to provide a liquid leaven composition that is stable and that may advantageously be stored for a longer period.

### Summary of the invention

The present invention is detailed in indepentent claims 1, 16 and 17 and in the thereof depending claims. It was surprisingly found that it is possible to combine or admix in a liquid formulation at least a bread flavour improvement composition, a bread improver composition and active yeast to obtain a liquid leaven composition which does not have the drawbacks of the prior art and which advantageously is stable.

As soon as there are traces of fermentable substrates introduced into a liquid yeast or leaven composition, a refermentation of the yeast can take place with instability during time as a consequence.

Some of the bread flavour improvement systems contain alcohols which could be a potential carbon source, but the product stayed stable anyhow.

Also the bread improver composition can be a source (N-source) for the yeast to start growing with both decreased activity of the improver and decreased gassing power of the yeast.

It was found that fermentable sugars, above a certain residual level, have a negative effect on the stability of a liquid leaven composition.

Especially the presence of flour, even the presence of flour traces, as source of fermentable components such as fermentable sugars can pose a problem for the stability of a liquid leaven composition.

The present invention relates to a process for producing a stable liquid leaven composition comprising the steps of admixing in a liquid formulation at least a flavour improvement composition, an improver composition and an active yeast, in such a way that the combined product is a stable liquid product having the combined properties of all three components separately.

Stability of the above product advantageously is obtained by keeping the residual sugar level of the liquid leaven composition low, to a minimum, below 0.5% w/w, more preferably below 0.4%, 0.3%, 0.2% or even below 0.1% w/w on the liquid leaven composition (the final product).

If needed, measures are taken to prevent a severe pH drop such as a drop of pH below 3.5 or below 4.0.

In the present invention a process is disclosed for the production of a stable liquid leaven composition, the process comprising the steps of:
- admixing in a liquid formulation at least a flavour improvement composition; a bread improve composition; and an active yeast, and
- ensuring that the residual sugar level of the liquid leaven composition is kept low, below 0.5% w/w on said liquid composition in order to obtain a stable liquid leaven composition.
Even more preferably the residual sugar level of the liquid leaven composition is kept below 0.4%, 0.3%, 0.2% or even below 0.1% w/w on said liquid composition.

A liquid formulation may be obtained by admixing the above ingredients with a liquid such as water and/or alcohols or glycerol for instance for enzyme stability. Alternatively, one may combine each of the above ingredients in liquid form.

Advantageously the flavour improvement composition is one that comprises at least one "sourdough or sponge based composition". As such a method or process is disclosed for producing a stable liquid leaven composition, the process comprising the steps of:
- admixing in a liquid formulation at least a flavour improvement composition comprising at least one sourdough or sponge based composition; a bread improver composition; and an active yeast, and
- ensuring that the residual sugar level of the liquid leaven composition is kept low, below 0.5% w/w on said liquid composition in order to obtain a stable liquid leaven composition.
Even more preferably the residual sugar level of the liquid leaven composition is kept below 0.4%, 0.3%, 0.2% or even below 0.1% w/w on said liquid composition.

By a "sourdough or sponge based composition" is meant that the flavour improvement composition comprises at least one of the following: a sourdough, a sourdough product, a sponge, a sponge product. The term "sourdough or sponge based composition" extends to a supernatant of a (liquid) sourdough, of a sourdough product, of a sponge, and/or of a sponge product. Preferably this supernatant is a concentrated supernatant.

Preferably the flavour improvement composition that is admixed comprises at least one of the following: a sourdough; a sourdough product ; a sponge; a sponge product ; a supernatant of a sourdough, of a sourdough product, of a sponge or of a sponge product.

The flavour improvement composition that is admixed may be a flour based improvement composition, a flavour improvement composition that comprises flour.

In a method according to the invention, the residual sugar level is kept low, below 0.5% w/w on the liquid leaven composition, by hydrolising the flour contained in said flavour improvement composition prior to a fermentation step to liberate fermentable sugars out of the starch. These liberated sugars are advantageously eliminated, or at least reduced to a residual sugar level below 0.5%, 0.4%, 0.3%, 0.2%, 0.1% w/w on the liquid leaven composition, by a microbial fermentation step. The microbial fermentation step advantageously further creates all the necessary flavour compounds.

Hydrolysing enzymes, such as an amylase, may be used to hydrolyze the flour.

In a method according to the invention, the residual sugar level is kept low, below 0.5% w/w on the liquid leaven composition, by using (admixing) a flavour improvement composition comprising at least one of the following: a supernatant of a liquid sourdough, a supernatant of a sourdough product, a supernatant of a sponge or a supernatant of a sponge product.

This supernatant may be obtained as follows: a typical yeast sponge dough or sourdough for instance is produced by fermenting flour slurry with yeast and/or lactic acid bacteria, whereafter a liquid supernatant is separated from the insoluble part. The liquid supernatant may advantageously be concentrated, for instance by a physical process known in the art. The liquid supernatant used (admixed) as flavour improvement composition advantageously is one with a residual sugar level below 0.5%, 0.4%, 0.3%, 0.2%, 0.1% w/w on the liquid leaven composition.

In a method according to the invention, the residual sugar level is kept low, below 0.5% w/w on the liquid leaven composition, by using (admixing) a sponge based flavour improvement composition. A sponge which contains ethanol in amounts up to 10% may be admixed provided that no flour traces remain in said sponge based flavour improvement composition.

In a method according to the invention (any of the above disclosed), the residual sugar level or the amount of fermentable sugars remaining in the final liquid leaven composition is advantageously kept to the minimum, below 0.5%, 0.4%, 0.3%, 0.2%, or 0.1% w/w on the final product (the final liquid leaven composition).

The bread improver composition used (admixed) may comprise chemical additives and/or enzymes.

Chemical additives may be selected from the group consisting of oxidizing/reducing agents such as ascorbic acid (example of an oxidising agent), cystein, gluthation (examples of reducing agents), hydrolysed gluten, yeast extracts, emulsifiers such as DATEM, SSL, CSL, GMS, bile salts, fatty materials and any mixture or blend thereof.

Enzymes may be selected from the group consisting of amylases, hemi-cellulases, oxidases, proteases, lipases and any mixture thereof.

Preferably fresh yeast is used (admixed).

The yeast that is admixed may be used under the form of compressed yeast with a dry matter of around 30% and/or may be used under the form of liquid yeast, preferably with a dry matter below 25%.

The liquid leaven composition according to the invention may be further stabilised by adding (admixing) a solution comprising a hydrocolloid or a gum, preferably a xanthane gum to the liquid leaven composition and/or by continuous mixing of the liquid leaven composition to prevent decantation.

Alternatively, the liquid leaven composition according to the invention may be further stabilised by using a 1% level of an exopolysaccharide such as a dextran in the final product (the liquid leaven composition of the invention) thereby preventing decantation.

In addition, a drop of the pH below 3.5 or below pH 4.0 may be prevented, for instance by adding a buffering system to the flavour improvement composition, by controlling the pH and/or by selecting specific bacterial strains, in particular specific lactic acid bacterial strains.

The invention further relates to a liquid leaven composition obtainable by a method according to the invention.

The obtained liquid leaven composition advantageously is stable.

The novel and inventive liquid leaven composition according to the invention preferably remains stable when stored for a longer period at low temperatures such as about 4°C. Preferably the product remains stable for at least one week at these temperatures, more preferably for at least about 4 weeks.

The present invention also relates to a dough, a bakery product, a pizza or a snack (e.g. crackers, pretzels, biscuits, ...) comprising the liquid leaven composition according to the present invention.

The present invention further concerns the use of the liquid leaven composition of the invention in the preparation process of a bakery product such as a bread, a pizza or a snack (e.g. crackers, pretzels, biscuits, ...).

### Brief description of the figures

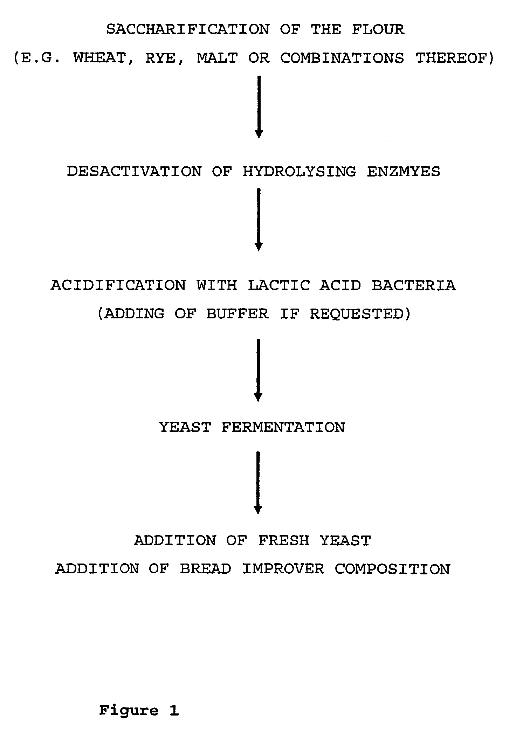

The figure 1 represents a schematic overview of a method for producing a stable liquid leaven composition according to the invention, wherein the amount of residual sugar is kept low.

The invention will be described in further details in the following examples by reference to the enclosed drawings.

### Detailed description of the invention

Throughout this specification the following terms and definitions are used:

By a "flavour improvement system", a "flavour improvement composition", a "bread flavour improvement system" or a "bread flavour improvement composition" is meant a sourdough or a sourdough product; a bakery sponge or a sponge product.

By a "sourdough" is meant a dough fermented by lactic acid bacteria and/or yeast, having a characteristic acidic flavour due to the lactic acid bacteria producing mainly lactic acid, acetic acid and some minor compounds and the typical flavour top-notes produced by the yeast.

A "sourdough product" in the present context refers to the product above, that is stabilized in one or another way (e.g. through drying, pasteurization, cooling, freezing, ...) so that this product can be added to a regular dough, thereby replacing the in-bakery produced prefermentation.

By a "sponge" or "sponge dough" is meant a dough fermented by yeast, having a characteristic flavour due to said yeast fermentation. It is a pre-fermentation product based on a yeast fermentation of part of the flour.

A "sponge product" refers to the stabilized form of such a regular bakery sponge fermentation, used to enhance the flavour in a regular dough. It can be a sponge extract.

"Improvement systems", "bread improvement systems", "improver compositions", or "bread improver compositions" may comprise chemical additives and/or enzymes, which are added to the dough in order to improve dough handling properties and/or to improve the quality of the final baked product. Chemical additives include but are not limited to oxidizing/reducing agents (ascorbic acid, cystein, gluthation etc), hydrolysed gluten, yeast extracts emulsifiers, fatty materials and others. There is a wide range of enzymes that are conventionally used for bread improvement purposes. These enzymes are all well known and described in literature.

"Liquid yeast" corresponds to a new trend in bakery and is active yeast having a dry matter up to 25%, and often stabilized with hydrocolloids (see e.g. EP-A-0461725).

The present invention relates to the production process for a liquid leaven composition that comprises at least a flavour improvement composition, a yeast and a bread improver composition and that improves the flavour of the final bread similarly to a bread made with the components of the improver composition each dosed separately. In the liquid leaven composition according to the present invention, the yeast is sufficiently stable and shows a gassing production capacity comparable to any regular liquid yeast. The bread improver composition in this novel and inventive composition is sufficiently stable and results in dough and/or bread improvement properties comparable to those obtained when the components are each dosed separately.

To ensure stability of the complex flavour and leaven system of the present invention, the amount of fermentable substrates (such as fermentable sugars) is kept below 0.5% w/w on the final product (the liquid leaven composition obtained). The liquid leaven composition according to the invention as such advantageously can be stored for at least one week, or even for at least about 4 weeks at about 4°C without any significant loss in activity or properties.

In sequel it is further explained that a normal freshly made sourdough based on regular flour without any processing could not be used as such because of flour components therein.

A sponge which contains ethanol in amounts up to 10% seemed not to be a problem for stability as long as there were no flour traces anymore. Flour can be regular wheat flour, but also other types of flour such as rye, malt, corn,... flour and mixtures thereof.

In the case that a sourdough is being used (admixed) in the liquid leaven composition, control of the pH may be needed.

Below, more details and some examples are given on how the problem of stability of the product (a liquid leaven composition) could be solved.

For the stability of the yeast within a (bread) flavour improvement composition or liquid leaven composition according to the invention, the residual sugar content should be as low as possible, below 0.5%, even better below 0.4%, 0.3%, 0.2% or even below 0.1% on the (final) liquid leaven composition.

Hereby a refermentation by the yeast in the packaging is prevented, which would otherwise result in severe foaming problems and cause instability of the yeast. Preferably the yeast in the composition according to the invention maintains more than 90% of its leavening activity when stored and handled properly.

The elimination of most fermentable sugars in for instance a sourdough product can be obtained in different ways.

Hydrolysis of the flour prior to fermentation is a first option. The fermentable sugars produced can then be consumed in a sourdough or sponge process generating all necessary flavour compounds and limiting residual (fermentable) sugars.

Alternatively on may use only the supernatant of a liquid sourdough for instance as flavour improvement composition, preventing hereby high amounts of starch into the liquid leaven composition. Minimal amounts of starch are present in the liquid phase (supernatant). Starch, if present, could be further hydrolysed by microbial activity during storage causing gas production and instability.

Another possibility is to reduce the residual sugar content (that of fermentable sugars) by using the same compositions as mentioned above, but based on a sponge dough. No acidic flavour profile will be produced in this case.

In order to stabilise the bread improver composition or liquid leaven composition according to the invention, the pH should not drop too much during sourdough production (i.e. the pH should preferably not drop below pH 3).

In the case that enzymes are being used, such as e.g. amylase, the buffer capacity should be optionally improved in the sourdough production preventing a severe drop in pH. The pH should in this case preferably not fall beneath pH 3.5, optimally not beneath pH 4, to prevent loss of activity of the bread improvement system. The use of special lactic acid bacteria not acidifying beneath pH 3.5 is another possibility. This can easily be checked by following the pH profile during acidification using a pH-meter. Residual sugars will at that time be consumed by the yeast present during flavour formation.

It was surprisingly found that when respecting the above conditions, i.e. by keeping the residual sugar content low, preferably below 0.5% w/w on the (final) liquid leaven composition and/or by preventing severe and undesired pH drops, a stable liquid leaven composition could be obtained showing properties comparable to those obtained when each of the components would have been dosed separately.

### Examples

In the examples, the following materials and methods were used.

A typical yeast sponge dough was produced by fermenting flour slurry with yeast during several hours. The liquid supernatant is separated from the insoluble part and concentrated by physical process. The residual sugar level is below 0.5%.

Alternatively, a sourdough product was produced using a special flour extraction method as shown in figure 1.

Ingredients of a (typical) bread improvement that was used:
Vitamin C : Ascorbic acid
Bel'ase A75: fungal amylase (BELDEM, Belgium)
Bel'ase B210: bacterial xylanase (BELDEM, Belgium)
Bel'ase XL1: phospholipase (BELDEM, Belgium)

Compressed fresh yeast with a 30% dry matter content or alternatively, liquid yeast with a dry matter content of max. 25% and kept in suspension by continuous mixing, was used.

### Example 1

Liquid sourdough was produced according to a scheme as presented in Figure 1. A first step comprised saccharification of the flour (wheat, rye, malt, ... or any combination thereof) following the here-described hydrolysis protocol.

*Hydrolysis protocol*: Flour, which can be regular wheat flour, but also other types of flour such as rye, malt, corn,... flour and mixtures of them are added to water to obtain a flour slurry. The optimal dry matter content of this slurry is between 20 and 50% and more particular between 30 and 35%. The slurry is then heated, optimally to temperatures between 75 and 95°C, more particular to a temperature about 90°C ± 2% and the pH is adjusted to a pH of 5.5 ± 0.5. The slurry is maintained under constant mixture. An amylase is added, e.g. Thermamyl SC^{®} (Novozymes, DK), at a dose of 0.4-0.5kg/ton of dry matter. Other amylases may be used. During the process of hydrolysis, the temperature is maintained at about 90°C and in the case of Thermamyl SC^{®}, the reaction time was about 90 minutes. The reaction time will be dependent on the substrate and enzymes used, and a person skilled in the art will know how to adapt the described protocol accordingly.

After this hydrolysis step, the temperature is lowered, the pH adjusted and a dextrinisation can take place adding a second enzyme cutting the oligodextrines into fermentable sugars. In this particular case, dextrozyme GA (0.8kg/T dry matter) (Novozymes, DK) was used at a temperature of about 60°C and at a pH of about 4.3. This second hydrolysis was done during 22 hours. Dependent on the substrate and the enzyme, these conditions can be adapted by the person skilled in the art.

After a step of hydrolysis and deactivation of the hydrolysing enzymes, an acidification step as described below was performed.

*Acidification step*: After saccharification of the flour, lactic acid bacteria are added. The slurry is diluted with water before fermentation. In this example, the slurry was diluted to obtain a sugar content of around 10%. This slurry is inoculated with a lactic acid bacterium (*Lactobacillus plantarum* sp) at a rate of 10 E+7/g of dry matter. The acidification takes place during about 24 hours at about 35°C. These fermentation conditions can be adapted according to the strain used and the flavour profile aiming for. Colder fermentation conditions generally will produce more acetic acid, whereas warmer temperatures more lactic acid.

To ferment residual sugars after the acidification step, some yeast is added. This yeast will consume the sugar to values lower then 0.5% w/w assuring the stability of the mixture afterwards.

After this step, the product is inactivated by heating the product, in the present case obtained by about 30 minutes heating at about 85°C.

To the above fermentation product, standard liquid yeast can now be added.

The composition of the bread improvement composition used in this first example is given in Table 1 below:

**Table 1**

| **Compound** | **Proportion** |
|---|---|
| Vitamin C | 5 |
| FRIMASE 210 (BELDEM, Belgium) | 3 |
| Amylase A75 (BELDEM, Belgium) | 1.5 |
| Bel'ase XL1 (BELDEM, Belgium) | 2.5 |
| Flour | 488 |
| Total | 500 |

The Dosage level used was 0.5% on total flour weight.

A bread was then baked following the recipe described below. In this first example, a comparison was made between a bread that that was prepared using the novel leaven composition of the invention (recipe 3) versus a bread whereby all the different components were dosed separately (recipes 1 and 2). Both the use of separately dosed compressed and liquid yeast was compared. Table 2 gives an overview of the components and their amounts used in the bread making process.

**Table 2 (grams or % on total flour weight)**

| **Recipe** | | **1** | **2** | **3** |
|---|---|---|---|---|
| Wheat flour | (25°) | 2000 | 2000 | 2000 |
| H₂O | ($) | 1100 | 1080 | 1080 |
| **Compressed yeast** | | **40** | **---** | **--** |
| **Liquid yeast** | | **---** | **66** | **--** |
| Salt | | 40 | 40 | 40 |
| **Bread improvement system** | (1) | **0.5%** | **0.5%** | |
| **Sourdough product** | | **1.65%** | **1.65%** | |
| **New leaven composition** | (2) | | | **5%** |

| | | | | |
|---|---|---|---|---|
| (1): The composition of the bread improver composition is given in table 1. (2): The composition of the liquid leaven composition according to the invention is given in Table 3 below. ($): The process conditions applied were those given in Table 4 below. | | | | |

**Table 3**

| | g/100kg flour |
|---|---|
| Bel'ase B 210 (BELDEM, Belgium) | 3.0 |
| Bel'ase A75 (BELDEM, Belgium) | 1.5 |
| Vitamin C | 5.0 |
| Bel4ase XL1 (BELDEM, Belgium) | 2.5 |
| Sourdough product based on rye flour | 1650 |
| Liquid Yeast | 3338 |
| Total | 5000.0 |

**Table 4**

| Mixing time: | 2'+4'30" | 2'+4'30" | 2'+4'30" |
|---|---|---|---|
| Eberhardt N24 | | | |
| Temperature - H₂O (°C) | 18° | 18° | 18° |
| Temperature - dough (°C) | 29.4° | 29.5° | 29.6° |

The following observations were made when the bread was prepared with a liquid leaven composition that was stored for 1 week before use (Table 5). A comparison was made with a bread made using conventional ingredients, i.e. with each of the components dosed separately (recipes 1 and 2). The rating/scoring was done by a technician skilled in the art.

Table 6 summarizes the results of a test performed with a liquid leaven composition comprising flavour composition, liquid yeast and improvement composition that was maintained at about 4°C for 4 weeks. After 4 weeks, it was baked again without loss of activity. Again a comparison was made with traditional recipes for bread making (recipes 1 and 2).

**Table 5**

| **Recipe** | | **1** | **2** | **3** |
|---|---|---|---|---|
| Aroma - product | | | | Fermented rye |
| Dough properties | | | ± idem 1 | ± idem 1 |
| Crumb colour | | 6.5 | 6.5 | 6.5 |
| Crumb structure | | 6.5 | 6.5 | 6.5 |
| Volume per 1 (ml) | 3x | 2975 | 2975 | 3025 |
| | % compared to liquid yeast | | | +2% |
| | % compared to compressed yeast | | 0% | +2% |
| Fermentation speed | | 2.4 | 2.6 | 2.5 |
| | F120' | 4.3 | 4.4 | 4.3 |
| | F180' / F niv. 6 | 173' | 169' | 171' |
| Height before baking (cm) | | 9.8 | 9.7 | 9.7 |
| **Aroma - bread** | | Fermented rye flavour | Fermented rye flavour | Fermented rye flavour |
| | | -> ok | -> ok | -> ok |
| pH - bread | | 5.8 | 5.8 | 5.8 |
| Acidity - bread | (ml NaOH 0.1N) | 2.8 | 2.7 | 2.9 |

**Table 6**

| **Recipe** | | **1** | **2** | **3** |
|---|---|---|---|---|
| Aroma - product | | | | Fermented rye |
| Dough properties | | | ± idem 1 | ± idem 1 |
| Crumb colour | | 6.5 | 6.5 | 6.5 |
| Crumb structure | | 6.5 | 6.5 | 6.5 |
| Volume per 1 ml | (x2) | 2950 | 2950 | 3000 |
| | % against liquid yeast | | | +2% |
| | % against compressed yeast | | 0% | +2% |
| Fermentation speed | F60' | 2.4 | 2.4 | 2.4 |
| | F120' | 4.2 | 4.4 | 4.3 |
| | F180' / F niv. 6 | 167' | 169' | 168' |
| Height before baking (cm) | | 10.1 | 10.2 | 10.3 |
| Aroma - bread | | Fermented rye | Fermented rye | Fermented rye |
| | | → OK | →± OK | → OK |
| pH - bread | | 5.6 | 5.6 | 5.7 |
| Acidity - bread | (ml NaOH 0.1N) | 3.2 | 3.1 | 3.1 |

From the above it can be derived that the novel liquid leaven composition is very stable even after storage of 4 weeks at about 4°C. Dough and bread properties remained unchanged in comparison with the separately dosed compounds (recipes 1 and 2). Independent of the fact whether compressed or liquid yeast was used, the leaven composition was found stable.

### Example 2:

In a second example, a bread was made according to two different recipes: one with each of the components dosed separately (recipe 1) and one with the novel liquid leaven composition (recipe 2) (Table 7).

**Table 7 (grams or % on total flour weight)**

| **Recipe** | | **1** | **2** |
|---|---|---|---|
| Wheat flour | (25°) | 2000 | 2000 |
| H₂O | ($) | 1120 | 1120 |
| **Yeast** | | **40** | **--** |
| Salt | | 40 | 40 |
| **Bread improvement system** | (1) | **0.5%** | |
| **Sponge extract** | | **2%** | |
| **New liquid leaven composition** | (3) | | **4%** |

The composition of the bread improvement system (1) is the same as described in Example 1 above.

The composition of the liquid leaven composition (3) used in recipe (2) is given in Table 8 below.

**Table 8**

| **Component** | **Proportion** |
|---|---|
| Vitamin C | 5 |
| Bel'ase B210 (BELDEM, Belgium) | 3 |
| Bel'ase A75 (BELDEM, Belgium) | 1.5 |
| Bel'ase XL1 (BELDEM, Belgium) | 2.5 |
| K₂HPO₄ | 16 |
| Sponge extract | 2000 |
| Yeast | 1972 |
| Total | 4000 |

The process conditions ($) that were applied are summarized in Table 9.

**Table 9**

| Mixing time : Eberhardt N24 | | 2'+4'30" | 2'+4'30" |
|---|---|---|---|
| Temperature - H₂O | (°C) | 18° | 18° |
| Temperature - dough | (°C) | 30.1° | 29.9° |

Again, the liquid leaven composition that was used in the bread making recipe 2 was used after 1 week, respectively 4 weeks of storage. The results are summarized in Tables 10 and 11 respectively.

**Table 10**

| **Recipe** | | **1** | **2** |
|---|---|---|---|
| Dough | | OK ± | idem 1 |
| Crumb colour | | 7.5 | 7.5 |
| Crumb structure | | 7 | 7 |
| Volume | | 2800 | 2775 |
| | | | -1% |
| Aroma | | Typical | Typical |
| | | Sponge | sponge |
| pH | | 5.7 | 5.7 |
| Acidity | (ml NaOH 0.1N) | 3.7 | 3.7 |

**Table 11**

| **Recipe** | | **1** | **2** |
|---|---|---|---|
| Dough | | OK ± | idem 1 |
| Crumb colour | | 7.5 | 7.5 |
| Crumb structure | | 7 | 7 |
| Volume | | 2800 | 2725 |
| | | | - 3% |
| Aroma | | Typical Sponge | Typical sponge |
| PH | | 5.7 | 5.7 |
| Acidity | (ml NaOH 0.1N) | 3.7 | 3.7 |

Also this example demonstrates that the liquid leaven composition according to the invention comprising a sponge extract, fresh yeast and a bread improver composition is stable for about 4 weeks at about 4°C.

## Claims

1. A process for stabilizing a liquid leaven composition comprising the steps of admixing, in a liquid formulation, at least:
- a flavour improvement composition that comprises at least one of the following:
- a sourdough, a sourdough product, a sponge, or a sponge product, wherein the flour contained in said flavour improvement composition is hydrolyzed prior to a fermentation step to liberate fermentable sugars out of the starch, these liberated sugars being eliminated by a microbial fermentation step;
- a supernatant of a sourdough, of a sourdough product, of a sponge or of a sponge product, wherein, optionally, the flour contained in said flavour improvement composition is hydrolyzed prior to a fermentation step to liberate fermentable sugars out of the starch, these liberated sugars being eliminated by a microbial fermentation step;
- a bread improver composition; and
- an active yeast;
wherein the residual sugar level of the liquid leaven composition is below 0.5% w/w on said liquid composition.

2. The process according to claim 1, wherein said flavour improvement composition comprises a supernatant of a liquid sourdough, a supernatant of a sourdough product, a supernatant of a sponge or a supernatant of a sponge product.

3. The process according to claim 2, wherein said supernatant is a concentrated supernatant.

4. The process according to any of claims 1 to 3, wherein a hydrolyzing enzyme, such as an amylase, is used to hydrolyze the flour.

5. The process according to any of claims 1 to 4, wherein said flavour improvement composition is a sponge based flavour improvement composition.

6. The process according to claim 5, wherein said sponge based flavour improvement composition contains up to 10% alcohols, provided that no flour traces remain.

7. The process according to any of the preceding claims, wherein said bread improver composition comprises chemical additives and/or enzymes.

8. The process according to claim 7, wherein said chemical additives are selected from the group consisting of oxidizing/reducing agents such as ascorbic acid, cystein, glutathione, yeast extracts, hydrolyzed gluten, emulsifiers such as DATEM, SSL, CSL, GMS, bile salts, fatty materials and any mixture thereof.

9. The process according to claim 8, wherein said enzymes are selected from the group consisting of amylases, hemi-cellulases, oxidases, proteases, lipases and any mixture thereof.

10. A process according to any of the preceding claims, wherein said active yeast is fresh yeast.

11. The process according to claim 10, wherein said fresh yeast is used under the form of compressed yeast with a dry matter of around 30% and/or under the form of liquid yeast, preferably with a dry matter below 25%.

12. The process according to any of the preceding claims, further comprising the step of adding a hydrocolloid or a gum, preferably a xanthan gum to the liquid leaven composition and/or the step of continuously mixing the liquid leaven composition to prevent decantation.

13. The process according to any of the claims 1 to 12, further comprising the step of adding 1% level of an exopolysaccharide such as a dextran in the final product thereby preventing decantation.

14. The process according to any of the preceding claims, further comprising the step of maintaining the pH above 3.5, preferably above 4.0.

15. The process according to claim 14, wherein the pH is maintained above 3.5, preferably above 4.0, by adding a buffering system to the flavour improvement composition, and/or by selecting specific lactic acid bacterial strains.

16. A liquid leaven composition obtainable by a method according to any of claime 1 to 15.

17. The use of the liquid leaven composition according to claim 16 in the preparation process of a bakery product such as a bread, a pizza or a snack.

## Patentansprüche

1. Verfahren zum Stabilisieren einer flüssigen Teigzusammensetzung, welches durch den Schritt des Mischens mit wenigstens einer der folgenden Zubereitungen erzielt wird:
- eine Geschmacksverbesserungszusammensetzung, welche mindestens eines aus dem Folgenden umfasst:
- einen Sauerteig, ein Sauerteigprodukt, einen Vorteig oder ein Vorteigprodukt , wobei das in der Geschmacksverbesserungszusammensetzung enthaltene Mehl vor der Fermentation hydrolysiert wird, um gärfähige Zucker aus der Stärke freizusetzen, die dann durch eine mikrobielle Fermentation eliminiert werden;
- einen Überstand eines Sauerteigs, Sauerteigprodukts, Vorteigs oder Vorteigprodukts , wobei das in der Geschmacksverbesserungszusammensetzung enthaltene Mehl vor einer Fermentation gegebenenfalls hydrolysiert wird, um gärfähige Zucker aus der Stärke freizusetzen, die dann durch eine mikrobielle Fermentation eliminiert werden;
- eine Brotverbesserungszusammensetzung und
- eine aktive Hefe;
wobei der Restzuckeranteil der flüssigen Teigzusammensetzung unter 5 % (w/w) liegt.

2. Verfahren nach Anspruch 1, wobei die Geschmacksverbesserungszusammensetzung den Überstand eines flüssigen Sauerteigs, den Überstand eines Sauerteigprodukts, den Überstand eines Vorteigs oder den Überstand eines Vorteigprodukts umfasst.

3. Verfahren nach Anspruch 2, wobei es sich bei dem Überstand um einen konzentrierten Überstand handelt.

4. Verfahren nach einem der Ansprüche 1 bis 3, wobei ein hydrolysierendes Enzym, z.B. eine Amylase, verwendet wird, um das Mehl zu hydrolysieren.

5. Verfahren nach einem der Ansprüche 1 bis 4, wobei es sich bei der Geschmacksverbesserungszusammensetzung um eine Geschmacksverbesserungszusammensetzung auf Vorteigbasis handelt.

6. Verfahren nach Anspruch 5, wobei die Geschmacksverbesserungszusammensetzung auf Vorteigbasis bis zu 10 % Alkohole enthält, vorausgesetzt, dass keine Mehlspuren zurückbleiben.

7. Verfahren nach einem der vorhergehenden Ansprüche, wobei die Brotverbesserungszusammensetzung chemische Additive und/oder Enzyme umfasst.

8. Verfahren nach Anspruch 7, wobei die chemischen Additive aus der Gruppe ausgewählt werden, die aus Oxidations-/Reduktionsmitteln wie Ascorbinsäure, Cystein, Glutathion, Hefeextrakten, hydrolysiertem Gluten, Emulgatoren wie DATEM, SSL, CSL, GMS, Gallensalzen, Fettstoffen und irgendeinem Gemisch daraus besteht.

9. Verfahren nach Anspruch 8, wobei die Enzyme aus der Gruppe ausgewählt werden, die aus Amylasen, Hemicellulasen, Oxidasen, Proteasen, Lipasen und irgendeinem Gemisch daraus besteht.

10. Verfahren nach einem der vorhergehenden Ansprüche, wobei es sich bei der aktiven Hefe um frische Hefe handelt.

11. Verfahren nach Anspruch 10, wobei die frische Hefe in Form von komprimierter Hefe mit einer Trockenmasse von ungefähr 30 % und/oder in Form von flüssiger Hefe, vorzugsweise mit einer Trockenmasse von weniger als 25%, verwendet wird.

12. Verfahren nach einem der vorhergehenden Ansprüche, welches ferner den Schritt der Zugabe eines Hydrokolloids oder eines Gummis, vorzugsweise eines Xanthangummis, zu der flüssigen Teigzusammensetzung und/oder den Schritt des kontinuierlichen Mischens der flüssigen Teigsammensetzung, um ein entmischen zu verhindern, umfasst.

13. Verfahren nach einem der Ansprüche 1 bis 12, welches ferner den Schritt der Zugabe eines Anteils von 1 % eines Exopolysaccharids wie Dextran in dem Endprodukt umfasst, wodurch ein entmischen verhindert wird.

14. Verfahren nach einem der vorhergehenden Ansprüche, welches ferner den Schritt des Haltens des pH-Wertes auf einem Wert von mehr als 3,5, vorzugsweise mehr als 4,0, umfasst.

15. Verfahren nach Anspruch 14, wobei der pH-Wert durch Zugabe eines Puffersystems zu der Geschmacksverbesserungszusammensetzung und/oder durch Auswahl spezieller Milchsäurebakterienstämme auf einem Wert von mehr als 3,5, vorzugsweise mehr als 4,0, gehalten wird.

16. Flüssige Teigzusammensetzung, die über ein Verfahren nach einem der Ansprüche 1 bis 15 zu erhalten ist.

17. Verwendung der flüssigen Teiglockerungsmittelzusammensetzung nach Anspruch 16 im Herstellungsverfahren für ein Bäckereiprodukt, z.B. ein Brot, eine Pizza oder einen Snack.

## Revendications

1. Procédé pour stabiliser une composition de levain liquide comprenant les étapes de mélange, dans une formulation liquide, d'au moins :
- une composition améliorant la flaveur qui comprend au moins un des éléments suivants :
- un levain, un produit de levain, une poolish ou un produit de poolish, où la farine contenue dans ladite composition améliorant la flaveur est hydrolysée avant une étape de fermentation pour libérer les sucres fermentescibles de l'amidon, ces sucres libérés étant éliminés par une étape de fermentation microbienne ;
- un surnageant d'un levain, d'un produit de levain, d'une poolish ou d'un produit de poolish, où facultativement la farine contenue dans ladite composition améliorant la flaveur est hydrolysée avant l'étape de fermentation pour libérer les sucres fermentescibles de l'amidon, ces sucres libérés étant éliminés par une étape de fermentation microbienne ;
- une composition améliorant le pain ; et
- une levure active ;
où le taux de sucre résiduel de la composition de levain liquide est inférieur à 0,5 % p/p sur ladite composition liquide.

2. Procédé selon la revendication 1, dans lequel ladite composition améliorant la flaveur comprend un surnageant d'un levain liquide, un surnageant d'un produit de levain, un surnageant d'une poolish ou un surnageant d'un produit de poolish.

3. Procédé selon la revendication 2, dans lequel ledit surnageant est un surnageant concentré.

4. Procédé selon l'une quelconque des revendications 1 à 3, dans lequel une hydrolase, telle qu'une amylase, est utilisée pour hydrolyser la farine.

5. Procédé selon l'une quelconque des revendications 1 à 4, dans lequel ladite composition améliorant la flaveur est une composition améliorant la flaveur à base de poolish.

6. Procédé selon la revendication 5, dans lequel ladite composition améliorant la flaveur à base de poolish contient jusqu'à 10 % d'alcools, à condition qu'aucune trace de farine ne reste.

7. Procédé selon l'une quelconque des revendications précédentes, dans lequel ladite composition améliorant le pain comprend des additifs chimiques et/ou des enzymes.

8. Procédé selon la revendication 7, dans lequel lesdits additifs chimiques sont choisis dans le groupe consistant en les agents oxydants/réducteurs tels que l'acide ascorbique, la cystéine, le glutathion, les extraits de levure, le gluten hydrolysé, les émulsifiants tels que DATEM, SSL, CSL, GMS, les sels biliaires, les matières grasses et tout mélange de ceux-ci.

9. Procédé selon la revendication 8, dans lequel lesdites enzymes sont choisies dans le groupe consistant en les amylases, les hémicellulases, les oxydases, les protéases, les lipases et tout mélange de celles-ci.

10. Procédé selon l'une quelconque des revendications précédentes, dans lequel ladite levure active est de la levure fraîche.

11. Procédé selon la revendication 10, dans lequel ladite levure fraîche est utilisée sous la forme de levure comprimée avec une matière sèche d'environ 30 % et/ou sous la forme d'une levure liquide, de préférence avec une matière sèche inférieure à 25 %.

12. Procédé selon l'une quelconque des revendications précédentes, comprenant en outre l'étape d'addition d'un hydrocolloide ou d'une gomme, de préférence d'une gomme de xanthane à la composition de levain liquide et/ou l'étape consistant à mélanger en continu la composition de levain liquide pour empêcher une décantation.

13. Procédé selon l'une quelconque des revendications 1 à 12, comprenant en outre l'étape d'addition d'un taux de 1 % d'un exopolysaccharide tel qu'un dextrane dans le produit final, pour empêcher ainsi une décantation.

14. Procédé selon l'une quelconque des revendications précédentes, comprenant en outre l'étape de maintien du pH au-dessus de 3,5, de préférence au-dessus de 4,0.

15. Procédé selon la revendication 14, dans lequel le pH est maintenu au-dessus de 3,5, de préférence au-dessus de 4,0, par ajout d'un système tampon à la composition améliorant la flaveur et/ou en sélectionnant des souches de bactéries lactiques spécifiques.

16. Composition de levain liquide pouvant être obtenue par un procédé selon l'une quelconque des revendications 1 à 15.

17. Utilisation de la composition de levain liquide selon la revendication 16 dans le procédé de préparation d'un produit de boulangerie tel qu'un pain, une pizza ou un en-cas.
